# EUROPEAN PATENT SPECIFICATION

(11) **EP 4 069 328 B1**
(45) Date of publication and mention of the grant of the patent: **29.10.2025**
(21) Application number: 20808351.9
(22) Date of filing: 17.11.2020
(51) Int. Cl.: A61L 31/06, A61L 31/02, A61L 31/14, A61F 2/07

(54) **METHOD FOR PRODUCING AN INTRALUMINAL ENDOPROSTHESIS WITH A BIODEGRADABLE SHEATH**
VERFAHREN ZUR HERSTELLUNG EINER INTRALUMINALEN ENDOPROTHESE MIT EINER BIOLOGISCH ABBAUBAREN HÜLLE
PROCÉDÉ DE FABRICATION D'UNE ENDOPROTHÈSE INTRALUMINALE À GAINE BIODÉGRADABLE

(30) Priority: 04.12.2019 DE 102019132938
(43) Date of publication of application: 12.10.2022
(73) Proprietor: Cortronik GmbH, 18119 Rostock-Warnemünde (DE)
(72) Inventor: KOHSE, Stefanie, 18059 Rostock (DE); LEBAHN, Kerstin, 18057 Rostock (DE); GRABOW, Niels, 18055 Rostock (DE); BAJER, Dalibor, 18147 Rostock (DE); GROSSMANN, Swen, 18055 Rostock (DE); SCHMITZ, Klaus-Peter, 18119 Rostock (DE); MUELLER, Heinz, 18055 Rostock (DE); MOMMA, Carsten, 18057 Rostock (DE)
(74) Representative: WSL Patentanwälte Partnerschaft mbB
(86) International application number: PCT/EP2020/082351
(87) International publication number: WO 2021/110407

(56) References cited:
- EP-A1- 1 974 756
- WO-A1-2018/156613
- WO-A2-2008/094971
- KR-A- 20180 090 881
- KR-B1- 101 198 464
- US-A1- 2018 104 044
- US-B2- 10 271 941

## Description

The invention relates to a method for producing an intraluminal endoprosthesis, in particular in the form of a stent.

Over the past 20 years, the number of interventional vascular (coronary and peripheral) procedures has been steadily increasing. At the same time, on the one hand increasingly more complex and difficult-to-access lesions are being treated, and on the other hand the number of older patients and patients with poor vascular substance (for example calcified and embrittled vessels) is increasing. This has led to an increasing number of patients whose vessels are injured during procedures by the catheters and guide wires used.

Perforations or ruptures of the treated vessels may occur. Perforations or ruptures are generally very serious, life-threatening complications, especially in coronary vessels, and must be treated immediately.

For such a treatment, for example in the coronary area, various so-called stent grafts are available. The coronary implants currently available consist of a permanent main body made of a metal (usually Co-Cr alloys) and a permanent polymer sheath, preferably made of PTFE or a polyurethane, which seals the damage in the vessel wall. This sheath can be a simple polymer tube or a fabric attached to the stent arranged therebeneath or thereabove.

One disadvantage of these implants is that they are only necessary until the vessel wall has healed sufficiently (approximately 2 - 3 days) to prevent blood from escaping through the perforated or ruptured area (haemostasis), but they then no longer perform any function.

However, permanent polymer sheaths (polymer tubes or spun coverings) in particular pose a major problem subsequently as they allow endothelialisation of the inner side of the vessel only very slowly and incompletely, thereby greatly increasing the risk of thrombosis or the risk of vessel occlusion in general.

KR 101198464 B1 discloses a drug-releasing coated stent. US 10,271,941 B2 describes implants with nonwoven fabrics. US 2018/0104044 A1 discloses closed cell stents. WO 2018/156613 A1 describes tracheal stents. Further, KR 2018/0090881 A1 describes a sustained release system. WO 2008/094971 A2 discloses a tubular tissue graft device. Moreover, EP 1 974 756 A1 discloses a stent comprising a citric acid ester.

In addition, normal vascular peristalsis is prevented by the usually very stiff implants. The main problems or complications are caused by the permanent polymer sheath for the above-mentioned reasons; the permanent support structure underneath (for example stent) poses a much smaller problem.

On this basis, the object of the invention is that of specifying an improved method for producing an intraluminal endoprosthesis which is improved in respect of one or more of the aforementioned problems.

This object is achieved by a method having the features of claim 1. A further aspect of the invention relates to an intraluminal endoprosthesis produced by means of the method according to claim 12.

Advantageous embodiments of these aspects of the invention are specified in the corresponding dependent claims and will be described below.

According to claim 1, a method for producing an intraluminal endoprosthesis is disclosed, in particular in the form of a stent, wherein the endoprosthesis comprises a support structure and a sheath arranged on the support structure, and wherein the method comprises the steps of:
- providing the support structure, and
   - forming the sheath (3) from polymer fibres (30) on the support structure (2), wherein a polymer solution (10) is dispensed from a nozzle (101) by means of electrospinning, wherein the polymer solution (10) comprises at least one biodegradable polymer and at least one additive,
**characterised in that**
the at least one biodegradable polymer is poly-L-lactide, the polymer solution (10) further comprising as additive 1,3-dioxan-2-one, with the proportion of 1,3-dioxan-2-one among the substances dissolved in the polymer solution (10) being in the range of 5 wt.% to 25 wt.%, based on the total mass of dissolved substances, the remaining proportion of the dissolved substances preferably being formed by poly-L-lactide.

Preferably, the sheath is spun so tightly that it is able to seal a vascular perforation or rupture against the blood pressure prevailing at the implantation site for at least 48 hours.

Support structures (especially stents), as well as the sheaths placed on them, are currently divided into two basic types: the permanent or durable support structures or sheaths and the biodegradable support structures or sheaths. Permanent support structures or sheaths are designed in such a way that they can remain in the vessel or at the implantation site in the human or animal body for an indefinite period of time. Biodegradable support structures or sheaths, on the other hand, are degraded in the vessel or body over a predetermined period of time. Preferably, biodegradable support structures are only broken down once the traumatised tissue of the vessel has healed and the support structure no longer needs to remain in the vessel lumen or body. Furthermore, it is preferable for a biodegradable sheath to be degraded only when it no longer needs to provide a sealing effect.

According to the method according to the invention, the at least one biodegradable polymer is poly-L-lactide

Furthermore, according to the method according to the invention, the polymer solution comprises at least one additive, wherein the at least one additive is 1,3-dioxan-2-one.

In general, the additive(s) substantially affect the mechanical properties (ductility) of the polymer fibres to ensure the necessary elongation at break. Here, the additive 1,3-dioxan-2-one can be used to lower the surface tension to improve the electrospinning process and the hydrophilicity of the cover.

Intraluminal endoprostheses can assume a compressed state in which they are inserted into the body at the implantation site. At the implantation site, the intraluminal endoprostheses are expanded and thus placed at the implantation site. Accordingly, the polymer fibre sheath must be able to withstand compression and expansion without detriment to the properties of the sheath, particularly in terms of permeability. The additives mentioned above improve the mechanical properties of the fibres of the sheath in a beneficial way. The mechanical properties of the polymer fibre sheath, particularly in terms of permeability, are maintained even in the event of compression and expansion. The elongation at break is increased or the surface tension is reduced so that the fibres are less susceptible to damage during compression or expansion.

If present as a component of the polymer solution, the at least one additive (except L-α-phosphatidylcholine) in the polymer solution has a concentration in the range of 5 wt.% to 25 wt.%, preferably 10 wt.% to 20 wt.%, particularly preferably 10 wt.% to 15 wt.%, based on the total mass of dissolved substances.

According to the method according to the invention, it is provided that the at least one biodegradable polymer is poly-L-lactide (CAS number 26161-42-2), the polymer solution further containing as additive 1,3-dioxan-2-one, wherein 1,3-dioxan-2-one accounts for a proportion among the substances dissolved in the polymer solution in the range of 5 wt.% to 25 wt.%, preferably 10 wt.% to 20 wt.%, particularly preferably 10 wt.% to 15 wt.%, preferably 12.5 wt.%, the remaining portion of the dissolved substances preferably being formed by poly-L-lactide. Other dissolved substances or substances otherwise present in the polymer solution may also be drugs and/or radiopaque substances.

Furthermore, according to an embodiment of the method, the polymer solution comprises a solvent, the solvent being selected from the group consisting of: chloroform (CHCl₃), trifluoroethanol (TFE), a mixture comprising chloroform and trifluoroethanol (TFE), with chloroform and trifluoroethanol preferably being present in a mixing ratio of 1:4 (chloroform:trifluoroethanol).

Furthermore, in accordance with an embodiment of the method, it is provided that the polymer solution has a concentration in the range of 1 wt.% to 10 wt.%, preferably 2 wt.% to 8 wt.%, particularly preferably 3 wt.% to 5 wt.%, preferably 4 wt.%, polymer (based on the mass of solvent(s) used).

All additives, drugs and/or radiopaque substances are added (polymer + additive(s) = 100% total mass of dissolved substances), the percentage being related to the total mass of dissolved substances and may lie between 0 and 20%. The amount of polymer in the solution remains constant.

The intraluminal endoprosthesis is a stent according to one embodiment, in particular a coronary stent or a peripheral stent.

The support structure, especially in the case of a stent, may be a self-expanding support structure or a balloon-expandable support structure. A balloon-expandable support structure may be produced in particular from a tube that is cut with a laser, for example. In the case of a self-expanding support structure, it may also be cut from a tube by laser cutting or, for example, may be formed from a suitable wire.

The support structure is preferably lattice-like and is formed by interconnected bars that define openings in the support structure. The bars or openings may be formed from a tube, for example, by laser cutting. A support structure made of a wire can also have a grid-like structure.

Furthermore, a support structure of interconnected bars defining openings can be formed from a non-metallic material (for example from a polymer, see also below).

Furthermore, according to an embodiment of the method, the support structure is a permanent support structure.

Such a permanent support structure may, for example, consist of one of the following materials or may comprise at least one of the following materials: a Co-Cr-based alloy, an Ni-based alloy, a corrosion-resistant stainless steel, an Ni-Ti alloy (with approximately equal atomic ratios of Ni and Ti, for example nitinol), optionally still containing less than 5% of one or more of the elements Co, Fe, Mn, a Ti-based alloy, an Nb-based alloy, a Ta-based alloy.

Furthermore, according to an alternative embodiment of the method, the support structure is a biodegradable metallic support structure.

Such a biodegradable metallic support structure may, for example consist of one of the following materials or may contain at least one of the following materials: an Mg alloy; an Mg-Al-Zn alloy; an Mg-Al-Mn alloy; an Mg-Al-Zn-Mn alloy; an Mg-RE alloy, wherein RE is selected from the rare earth group; an Mg-Y-RE alloy, wherein RE is selected from the rare earth group; an Mg-RE-Zn alloy, wherein RE is selected from the rare earth group; an Mg-Al-Y alloy; an Mg-Al-RE alloy, wherein RE is selected from the rare earth group; an Mg-Zn-Zr alloy, an Mg-Ca-Zn alloy; an Mg-Al alloy with an A1 content of 3 wt.% to 11 wt.%; an Mg-Ca-Zn alloy with a Zn content of 0.01 wt.% to 12 wt.%, preferably from 0.1 wt.% to 5 wt.%, and a Ca content of 0.01 wt.% to 5 wt.%, preferably from 0.1 wt.% to 1 wt.%; an Mg-Y-RE alloy, wherein RE stands for other rare earths (not Y), with a Y content of 0.1 wt.% to 5 wt.%, an Nd content of 0.01 wt.% to 5 wt.%, a Gd content of 0.01 wt.% to 3 wt.%, a Dy content of 0.01 wt.% to 3 wt.%, and wherein the alloy optionally comprises 0.1 wt.% to 1 wt.% Zr and other rare earths.

According to an alternative embodiment of the method according to the invention, the support structure is a biodegradable polymeric support structure. Here, balloon-expandable biodegradable polymeric stent scaffolds or support structures, which according to the present invention are provided with a biodegradable sheath, may have the stent designs described in DE 10 2016 117 398.

Furthermore, according to one embodiment of the method, it is provided that said polymeric support structure comprises one of the following materials or consists of one of the following materials: a biodegradable polymer; a poly-L-lactide; a poly-D,L-lactide; a poly-L-lactide-co-D,L-lactide; a polyglycolide; a polyanhydride; a polyhydroxybutyrate; a polyhydroxyvalerate; a poly-ε-caprolactone; a polydioxanone; a poly(lactide-co-glycolide); a poly(lactide-co-caprolactone); a poly(ethylene glycol-co-caprolactone); a poly(glycolide-co-caprolactone); a poly(hydroxybutyrate-co-valerate); a polytrimethylene carbonate-based polymer; a polypropylene succinate; a polyphosphazene; a poly-D,L-lactide-co-glycolide having a lactide content of 5 wt.% to 85 wt.%, preferably from 50 wt.% to 85 wt.%

According to an embodiment of the method, the material from which the support structure is formed or which the support structure comprises may also be a copolymer comprising two or more different monomers of the polymers of the above-mentioned group of materials.

Furthermore, the material from which the support structure is formed or which the support structure comprises may be, according to an embodiment, a copolymer or a blend of the above-mentioned polymers, the blend comprising two or more different polymers of the above-mentioned group of support structure materials.

Furthermore, the support structure may also comprise one of the following materials or may be formed from one of the following materials: a copolymer comprising hydroxybutyrate; a copolymer comprising valerate.

Furthermore, according to the method, it is provided that, during electrospinning, the polymer solution is applied from a nozzle to the support structure in fibre form.

The polymer solution is dispensed from the nozzle in accordance with an embodiment of the method, preferably with a volume flow in the range from 0.1 ml/h [millilitres per hour] to 2 ml/h, preferably 0.5 ml/h to 0.9 ml/h, preferably 0.7 ml/h.

According to an embodiment of the method, the nozzle preferably has a distance to the support structure which is in the range of 50 mm to 200 mm, preferably 70 mm to 110 mm, and preferably 90 mm.

Furthermore, according to an embodiment of the method, it is preferably provided that, between the nozzle (emitter) and a collector (mandrel), on which the support structure is arranged, there is applied an electrical voltage in the range of at least 1 kV, preferably 1 kV to 20 kV, particularly preferably 2 kV to 10 kV, preferably 4 kV.

It is expedient to place the intraluminal endoprosthesis on a mandrel during the electrospinning and to weave the sheath beyond the actual ends of the support structure consisting of individual connected bars. The longitudinal extent of the sheath spun from polymer fibres thus exceeds the longitudinal extent (length) of the support structure. The endoprosthesis with the excessively long polymer fibre sheath is then transferred to a suitable mandrel and trimmed by laser. In doing so, at most half the width of the outer ring segment of the support structure is no longer covered by the polymer fibre sheath. Such laser trimming of the polymer sheath prevents the removal of individual fibres or fibre flaps from the endoprosthesis.

Furthermore, in accordance with an embodiment of the method, it is provided that the electrospun sheath is exposed to a predefined temperature over a predefined period of time after the electrospinning, the period of time preferably being in the range of 10 h to 15 h, and the period of time preferably being 13.5 h, and the temperature preferably being in the range of 70°C to 90°C, preferably 80°C.

According to one embodiment of the method, the polymer solution may contain at least one drug, so that the electrospun sheath comprises and can release the at least one drug.

According to one embodiment of the method, the support structure, in particular a coating of the support structure, may comprise or contain a drug so that the drug can be released from the support structure/coating.

Such a drug incorporated into the polymer sheath or a support structure can be eluted from the polymer or a carrier material (for example coating of the support structure) preferably over a period of 7 days to 4 years and serves to achieve supportive effects for the therapy; such effects can be, among others:
- suppression of inflammatory processes that can occur especially during the degradation of bioresorbable materials,
- suppression of proliferative processes that can occur as a result of vascular injury,
- support for endothelialisation, and
- antithrombotic effects.

The at least one drug of the sheath and/or the support structure may be one of the following drugs according to an embodiment of the invention: a drug with antiproliferative activity, a drug with anti-inflammatory activity, a drug with antithrombotic activity, paclitaxel, everolimus, mycophenolic acid, angiopeptin, enoxaparin, hirudin, acetylsalicylic acid, dexamethasone, rifampicin, minocycline, budesonide, desonide, corticosterone, cortisone, hydrocortisone, prednisolone, heparin, heparin derivatives, urokinase, PPACK.

Furthermore, according to one embodiment of the method, the polymer solution comprises a substance visible under X-ray.

A further aspect of the invention relates to an intraluminal endoprosthesis, in particular in the form of a stent (for example coronary or peripheral stent), produced by the method according to the invention.

Embodiments as well as further features and advantages of the invention will be explained hereinafter with reference to the drawings, in which:
- Fig. 1: shows a schematic representation of an embodiment of the method according to the invention;
- Fig. 2: shows images recorded by scanning electron microscope of an intraluminal endoprosthesis produced by means of the method according to the invention, which endoprosthesis has a support structure (stent) made of a metal alloy and a biodegradable electrospun polymer sheath;
- Fig. 3: shows an embodiment of an intraluminal endoprosthesis according to the invention (right: support structure of the endoprosthesis in the form of a polymer stent; centre: electrospun sheath, left: support structure spun with sheath);
- Fig. 4: shows the elongation at break of an electrospun sheath in accordance with the invention in the form of a PLLA fleece (left) compared to a PLLA L210 (poly-L-lactide) foil (right);
- Fig. 5: shows an embodiment of an intraluminal endoprosthesis according to the invention comprising a biodegradable metallic support structure (in particular metal stent framework) with a biodegradable electrospun sheath before and after an expansion process;
- Fig. 6: shows a representation of the resulting permeability as a function of time of envelopes produced by means of the method according to the invention;
- Fig. 7: shows a laser-cut sheath which has been electrospun in accordance with the invention;
- Fig. 8: shows an additional laser-cut sheath which has been electrospun in accordance with the invention; and
- Fig. 9A to 9D: show images recorded by scanning electron microscope of polymer fibres of sheaths produced in accordance with the invention.

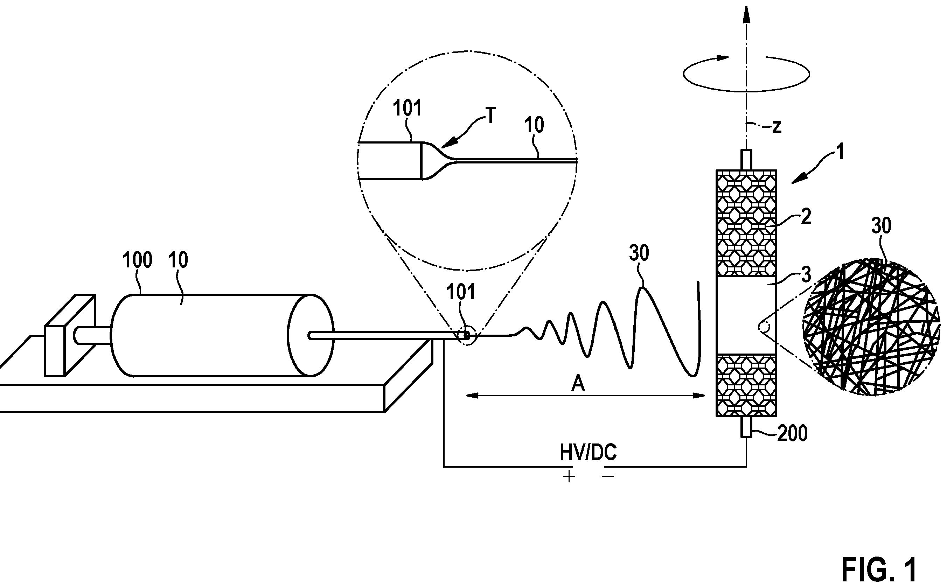

Figure 1 shows an embodiment of a method according to the invention for producing an intraluminal endoprosthesis 1, in particular in the form of a stent (stent graft), wherein the endoprosthesis 1 has a support structure 2 and a sheath 3 arranged on the support structure 2, and wherein the method comprises the steps of: providing the support structure 2, and forming the sheath 3 from polymer fibres 30 on an outer side of the support structure 2, wherein a polymer solution 10 is dispensed from a nozzle 101 by means of electrospinning, wherein the polymer solution 10 comprises at least one biodegradable polymer.

In electrospinning the polymer solution 10 is dispensed from a nozzle (for example at the end of a capillary), which is fluidically connected to a (for example syringe-like) reservoir 100, in which the polymer solution 10 is stored.

Here, an electrical voltage is applied between the nozzle 101, also known as an emitter, and a collector 200, which voltage may be in the range of 4 kV to 8 kV, for example. The support structure 2 is arranged on the collector 200 and can be rotated (for example by means of the collector 200) about a longitudinal axis z and, in particular, may be moved along the longitudinal axis z in order to distribute the polymer solution 10 in fibre form on the outside of the support structure 2.

In electrospinning, the polymer solution forms a so-called Taylor cone T due to said voltage at the nozzle 101 or capillary opening (see Figure 1), so that the polymer solution 10 is deposited in a disordered manner as a longitudinally stretched fibre 30 on the rotating support structure 2, which is also subjected to a high voltage. After evaporation of the solvent (escape of the solvent), a sheath 3 of fibres 30 is formed on the support structure 2 and these are bonded (glued) together to form a non-woven structure as a result of the time-resolved curing process.

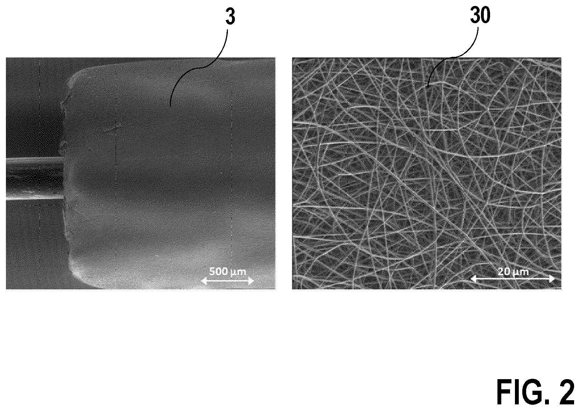

Figure 2 shows an image recorded by scanning electron microscope of an intraluminal endoprosthesis 1 produced by means of the method according to the invention, which endoprosthesis has a support structure (stent) 2 made of a Co-Cr alloy (L605) and a biodegradable electrospun polymer sheath 3.

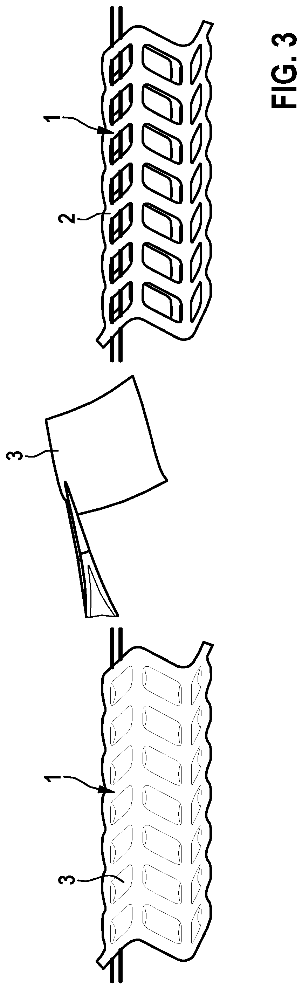

Figure 3 shows a further example according to the invention in the form of a biodegradable polymeric poly-L-lactide-based support structure or stent, which is covered by means of the method according to the invention with a sheath 3 in the form of a fibre nonwoven structure made of poly-L-lactide via electrospinning. The sheath 3 is hereby applied to the support structure 2 in the non-expanded state of the support structure 2. The desired layer thickness of the sheath 3 can be advantageously small and lies in the range of 10 µm to 100 µm, preferably 40 µm to 80 µm, especially preferably 50 µm to 70 µm.

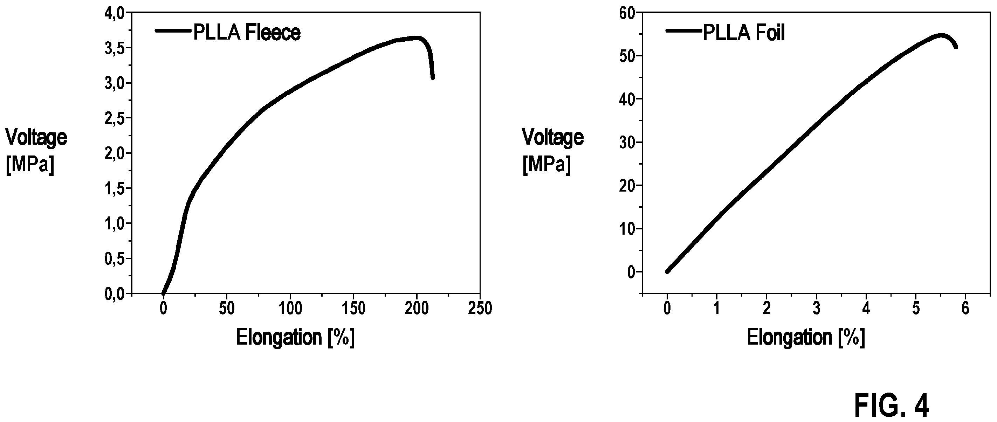

Due to the special morphology of the fibre nonwoven structures, an increased elongation at break of polymeric materials is achieved, so that the spectrum of usable polymers is advantageously increased. In this regard, Figure 4 shows the elongation at break of an electrospun fleece made of poly-L-lactide (left) compared to a poly-L-lactide foil (right).

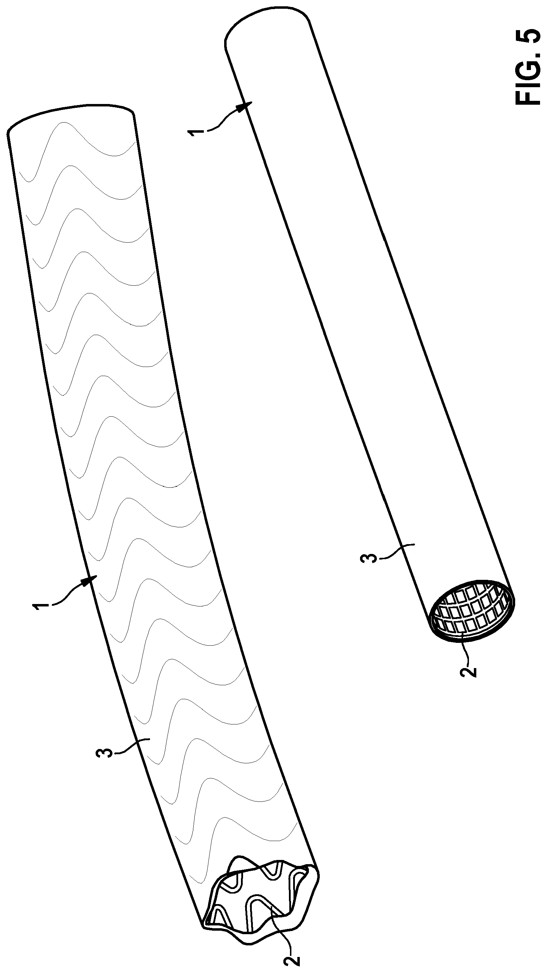

As a further example of an endoprosthesis produced in accordance with the invention, Figure 5 shows a biodegradable metallic Mg-based support structure (high-purity Mg-6.25%Al alloy), which has a biodegradable polymeric fibre nonwoven cover made of poly-L-lactide produced by electrospinning.

The covering of the support structure 2 with polymer solution 10 is carried out in the non-expanded state of the support structure 2. The achievable layer thickness can again be advantageously low (see above).

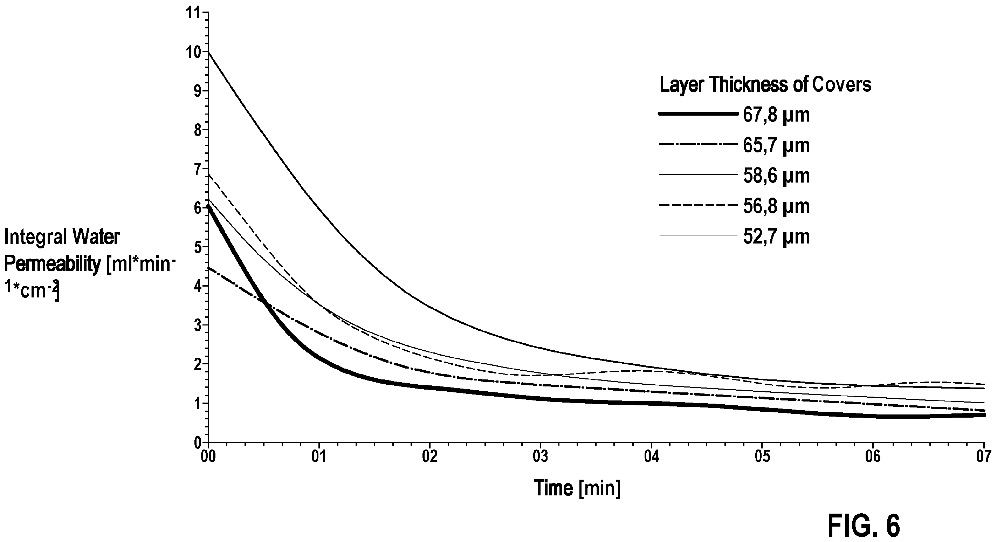

Furthermore, the invention also advantageously allows the use of polymers which allow a high diffusion of water into the polymer material, so that small layer thicknesses of the sheath with sufficient sealing effect can be achieved. Figure 6 shows the integral water permeability over time for 5 electrospun degradable polymer sheaths consisting of poly-L-lactide L210 (+12.5% dioxanone) with different layer thicknesses. The integral water permeability decreases in the beginning and converges after 5 minutes to a value below 2 ml*min⁻¹*cm⁻² for all layer thicknesses. This shows that with the present invention the desired low permeability can also be achieved with a low layer thickness of the polymer fibre sheath.

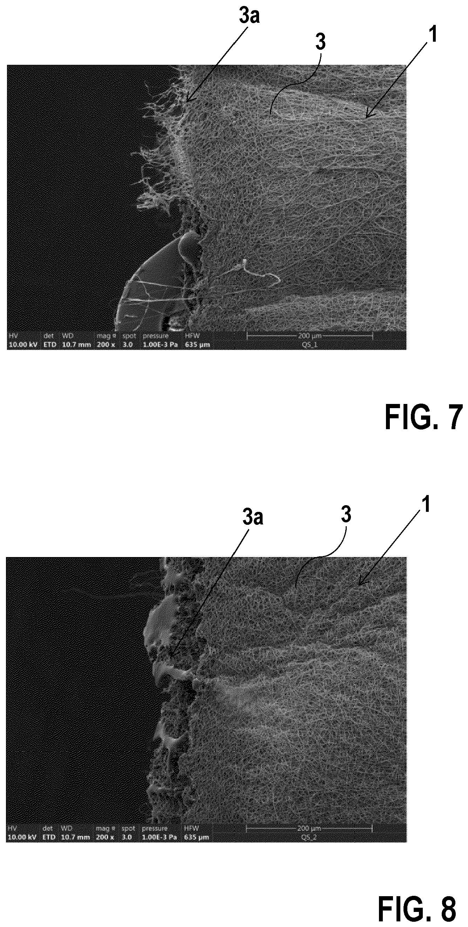

Figures 7 to 9D show further examples of intraluminal endoprostheses 1 produced in accordance with the invention, wherein the support structure (not shown) is made of a Co-Cr alloy and the polymer solution 10 is used as a biodegradable polymer or dissolved substance poly-L-lactide (PLLA L210, CAS number 26161-42-2) as well as 12.5 wt.% 1,3-dioxan-2-one (based on the totality of dissolved substances), wherein the polymer in the solvent (CHCl3:TFE, 1:4) has a concentration of 4 wt.%.

With these endoprostheses 1, the electrospinning process takes about 10 minutes per sheath 3. The support structures are each placed centrally on a mandrel of about 10 cm length, which forms the collector 200, with the collector having a travel of about 6 cm. The distance A between nozzle 101 and support structure 2 is 90 mm, wherein the polymer solution 10 is dispensed via the nozzle 101 with a volume flow of 0.7 ml/h [millilitres per hour] and is accelerated onto the support structure by means of an electrical voltage (for example the nozzle/emitter 101 can be at an electrical potential of 8 kV and the collector 200 at an electrical potential of 4 kV). During electrospinning, the temperature, air humidity, exhaust air, mass and layer thickness (for example by means of REM) are documented for comparison purposes. As a post-treatment, each prosthesis 1 is subjected to a tempering (for example for 13.5 h at 80°C). The produced sheaths 3 were trimmed using a CO₂ laser. The cut edges (see Figures 7 and 8) show only small fusions, but not with the supporting framework below. Furthermore, there is no two-dimensional fusion along the cut in the circumferential direction of the particular sheath 3.

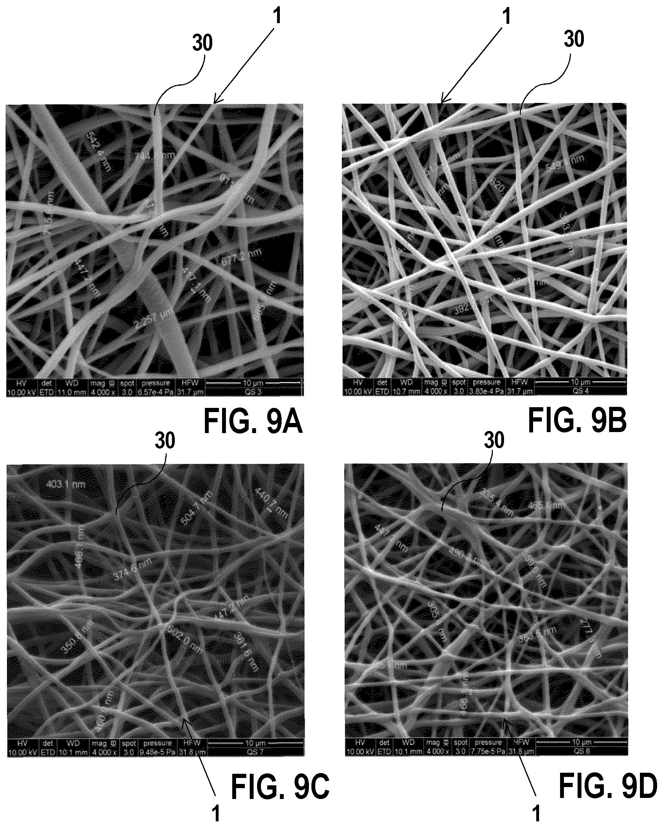

Figures 9A to 9D show exemplary diameters of the fibres 30, which are obtained by the electrospinning of the sheaths 3. The mean values of the diameters of the fibres 30 are 785 nm (Fig. 9A), 469 nm (Fig. 9B), 441 nm (Fig. 9C) and 419 nm (Fig. 9D).

Finally, a measurement report of an endoprosthesis 1 manufactured as described above is given as an example. The support structure 2 (stent) made of a Co-Cr alloy (L605) was weighed in the clean room before spinning (according to Figure 1) (mass m_{Stent, uncovered} = 10.28 µg). The stent 2 was then tested on the Diames test bench at IIB e.V. (test laboratory, Rostock-Warnemünde) with a laser to obtain the diameter; this is d_{Stent, uncovered, mean value} = 1.74 mm. Afterwards, the stent 2 was electrospun for 10 min and after the electrospinning process it was tempered for 13.5 h at 80°C in a vacuum drying oven. After tempering, the mass was again determined to m_{Stent, covered} = 11.63 µg. The diameter of the covered stent 2 was then determined again, d_{Stent, covered, mean value} = 1.88 mm, resulting in a layer thickness of the cover (sheath) 3 of 0.07 mm.

The covered stent 2 was widened (expanded) by placing it on a catheter, crimping it, then holding it in tempered water (37°C) for 30 seconds according to the test instructions, widening it to 3.0 mm within 30 seconds and leaving it in water for another 30 seconds (holding time). The expanded, covered stent was then removed from the catheter and dried once more at 80°C for 13.5 h in a vacuum drying oven. Finally, the covered stent 2 was sputtered onto a SEM slide and the fibre diameter was determined (see Figures 9A to 9D).

Raw data:
mass of the cover 3 (M_{Cover}): m_{Stent, uncovered} = 10.28 mg, m_{Stent}, covered = 11.63 mg, M_{Cover} = (11.63 - 10.28) mg = 1.35 mg
layer thickness of the cover 3: D_{Stent, covered} - D_{Stent, uncovered} = (1.88 - 1.74) mm = 0.14 mm,
layer thickness = (0.14 mm/2) = 0.07 mm.

The method according to the invention allows the production of a completely or partially biodegradable implant with an electrospun sheath for the treatment of vascular perforations and vascular ruptures.

The use of biodegradable polymers minimises potential late effects when using the implant through its degradation. The use of fibre nonwoven structures, which are created by using electrospinning, also offers the advantage that the spectrum of usable polymers is increased by the increased elongation at break.

The invention further allows the use of a sheath of reduced layer thickness with sufficient sealing by using polymer materials which allow a high diffusion of water into the polymer. Optionally, the polymer sheath can be supported by a rapidly swellable hydrogel.

## Claims

1. Method for producing an intraluminal endoprosthesis (1), wherein the endoprosthesis (1) comprises a support structure (2) and a sheath (3) arranged on the support structure (2), and wherein the method comprises the steps of:
- providing the support structure (2), and
- forming the sheath (3) from polymer fibres (30) on the support structure (2), wherein a polymer solution (10) is dispensed from a nozzle (101) by means of electrospinning, wherein the polymer solution (10) comprises at least one biodegradable polymer and at least one additive,
**characterised in that**
the at least one biodegradable polymer is poly-L-lactide, the polymer solution (10) further comprising as additive 1,3-dioxan-2-one, with the proportion of 1,3-dioxan-2-one among the substances dissolved in the polymer solution (10) being in the range of 5 wt.% to 25 wt.%, based on the total mass of dissolved substances, the remaining proportion of the dissolved substances preferably being formed by poly-L-lactide.

2. Method according to claim 1, **characterised in that** the intraluminal endoprosthesis (1) is a stent.

3. Method according to one of the preceding claims, **characterised in that** the at least one biodegradable polymer is poly-L-lactide, the polymer solution (10) further comprising as additive 1,3-dioxan-2-one, with the proportion of 1,3-dioxan-2-one among the substances dissolved in the polymer solution (10) being in the range of 10 wt.% to 20 wt.%, preferably 10 wt.% to 15 wt.%, based on the total mass of dissolved substances, the remaining proportion of the dissolved substances preferably being formed by poly-L-lactide.

4. Method according to claim 3, **characterised in that** the dissolved substances together in the polymer solution (10) have a concentration in the range from 1 wt.% to 10 wt.%, preferably 2 wt.% to 8 wt.%, particularly preferably 3 wt.% to 5 wt.%, preferably 4 wt.%.

5. Method according to one of the preceding claims, **characterised in that** the polymer solution (10) comprises a solvent, the solvent being selected from the group consisting of: chloroform (CHCl₃), trifluoroethanol (TFE), a mixture comprising chloroform and trifluoroethanol, with chloroform and trifluoroethanol preferably being present in a mixing ratio of 1:4.

6. Method according to one of the preceding claims, **characterised in that** the support structure (2) is a permanent support structure, preferably consisting of one of the following materials or comprising at least one of the following materials: a Co-Cr-based alloy, an Ni-based alloy, a corrosion-resistant stainless steel, an Ni-Ti alloy, a Ti-based alloy, an Nb-based alloy, a Ta-based alloy.

7. Method according to one of claims 1 to 6, **characterised in that** the support structure (2) is a biodegradable metallic support structure.

8. Method according to claims 1 to 5 or 7, **characterised in that** the support structure (2) comprises one of the following materials or is formed from one of the following materials: an Mg alloy; an Mg-Al-Zn alloy; an Mg-Al-Mn alloy; an Mg-Al-Zn-Mn alloy; an Mg-RE alloy, with RE being selected from the rare earth group; an Mg-Y-RE alloy, with RE being selected from the rare earth group; an Mg-RE-Zn alloy, with RE being selected from the rare earth group, an Mg-Al-Y alloy; an Mg-Al-RE alloy, with RE being selected from the rare earth group; an Mg-Zn-Zr alloy, an Mg-Ca-Zn alloy; an Mg-Al alloy with an Al content of 0.01 wt.% to 12 wt.%, preferably from 0.1 wt.% to 5 wt.%, and a Ca content of 0.01 wt.% to 5 wt.%, preferably from 0.1 wt.% to 1 wt.%; an Mg-Y-RE alloy, with RE standing for other rare earths different from Y, with a Y content of 0.1 wt.% to 5 wt.%, an Nd content from 0.01 wt.% to 5 wt.%, a Gd content from 0.01 wt.% to 3 wt.%, a Dy content from 0.01 wt.% to 3 wt.%, and with the alloy optionally comprising from 0.1 wt.% to 1 wt.% Zr and other rare earths.

9. Method according to one of claims 1 to 5, **characterised in that** the support structure (2) is a biodegradable polymeric support structure, preferably comprising one of the following materials or being formed from one of the following materials: a biodegradable polymer; a poly-L-lactide; a poly-D,L-lactide; a poly-L-lactide-coD,L-lactide; a polyglycolide; a polyanhydride; a polyhydroxybutyrate; a polyhydroxyvalerate; a poly-ε-caprolactone; a polydioxanone; a poly(lactide-co-glycolide); a poly(lactide-co-caprolactone); a poly(ethylene glycol-co-caprolactone); a poly(glycolide-co-caprolactone); a poly(hydroxybutyrate-co-valerate); a polytrimethylene carbonate-based polymer; a polypropylene succinate; a polyphosphazene; a poly-D,L-lactide-co-glycolide having a lactide content of 5 wt.% to 85 wt.%, preferably from 50 wt.% to 85 wt.%.

10. Method according to one of the preceding claims, **characterised in that** the polymer solution (10) is dispensed from a nozzle (101) during the electrospinning onto the support structure (2) in fibre form, preferably with a volume flow of the polymer solution (10) in the range of 0.5 mL/h to 0.9 mL/h, preferably 0.7 mL/h, the nozzle (101) preferably having a distance (A) from the support structure (2) in the range of 70 mm to 110 mm, preferably 90 mm, and an electrical voltage of at least 1 kV, preferably 1 kV to 20 kV, particularly preferably 2 kV to 10 kV, preferably 4 kV, preferably being applied between the nozzle (2) and a collector (200) on which the support structure (2) is arranged.

11. Method according to one of the preceding claims, **characterised in that** after electrospinning the sheath (3) is tempered at a predefined temperature for a predefined period of time, the period of time preferably being in the range of 10 h to 15 h, the period of time preferably being 13.5 h, and the temperature preferably being in the range of 70°C to 90°C, the temperature preferably being 80°C.

12. Intraluminal endoprosthesis (1) produced by the method according to one of the preceding claims.

## Patentansprüche

1. Verfahren zur Herstellung einer intraluminalen Endoprothese (1), wobei die Endoprothese (1) eine Stützstruktur (2) und eine auf der Stützstruktur (2) angeordnete Hülle (3) umfasst, und wobei das Verfahren die folgenden Schritte umfasst:
- Bereitstellen der Stützstruktur (2) und
- Ausbilden der Hülle (3) aus Polymerfasern (30) auf der Stützstruktur (2), wobei eine Polymerlösung (10) mittels Elektrospinnen aus einer Düse (101) abgegeben wird, wobei die Polymerlösung (10) mindestens ein biologisch abbaubares Polymer und mindestens ein Additiv umfasst,
**dadurch gekennzeichnet, dass**
das mindestens eine biologisch abbaubare Polymer Poly-L-Lactid ist, wobei die Polymerlösung (10) ferner als Additiv 1,3-Dioxan-2-on enthält, wobei der Anteil an 1,3-Dioxan-2-on an den in der Polymerlösung (10) gelösten Stoffen im Bereich von 5 Gew.-% bis 25 Gew.-%, bezogen auf die Gesamtmasse der gelösten Stoffe, liegt, wobei der verbleibende Anteil der gelösten Stoffe vorzugsweise durch Poly-L-lactid gebildet wird.

2. Verfahren nach Anspruch 1, **dadurch gekennzeichnet, dass** die intraluminale Endoprothese (1) ein Stent ist.

3. Verfahren nach einem der vorhergehenden Ansprüche, **dadurch gekennzeichnet, dass** das mindestens eine biologisch abbaubare Polymer Poly-L-lactid ist, wobei die Polymerlösung (10) als Additiv 1,3-Dioxan-2-on enthält, wobei der Anteil an 1,3-Dioxan-2-on unter den in der Polymerlösung (10) gelösten Substanzen im Bereich von 10 Gew.-% bis 20 Gew.-%, vorzugsweise 10 Gew.-% bis 15 Gew.-%, bezogen auf die Gesamtmasse der gelösten Substanzen, wobei der verbleibende Anteil der gelösten Substanzen vorzugsweise durch Poly-L-Lactid gebildet wird.

4. Verfahren nach Anspruch 3, **dadurch gekennzeichnet, dass** die gelösten Substanzen zusammen in der Polymerlösung (10) eine Konzentration im Bereich von 1 Gew.-% bis 10 Gew.-%, vorzugsweise 2 Gew.-% bis 8 Gew.-%, besonders bevorzugt 3 Gew.-% bis 5 Gew.-%, vorzugsweise 4 Gew.-%, aufweisen.

5. Verfahren nach einem der vorhergehenden Ansprüche, **dadurch gekennzeichnet, dass** die Polymerlösung (10) ein Lösungsmittel umfasst, wobei das Lösungsmittel ausgewählt ist aus der Gruppe bestehend aus: Chloroform (CHCl₃), Trifluorethanol (TFE), einer Mischung aus Chloroform und Trifluorethanol, wobei Chloroform und Trifluorethanol vorzugsweise in einem Mischungsverhältnis von 1:4 vorliegen.

6. Verfahren nach einem der vorhergehenden Ansprüche, **dadurch gekennzeichnet, dass** die Stützstruktur (2) eine permanente Stützstruktur ist, die vorzugsweise aus einem der folgenden Materialien besteht oder mindestens eines der folgenden Materialien umfasst: eine Co-Cr-basierte Legierung, eine Ni-basierte Legierung, einen korrosionsbeständigen Edelstahl, eine Ni-Ti-Legierung, eine Ti-basierte Legierung, eine Nb-basierte Legierung, eine Ta-basierte Legierung.

7. Verfahren nach einem der Ansprüche 1 bis 6, **dadurch gekennzeichnet, dass** die Stützstruktur (2) eine biologisch abbaubare metallische Stützstruktur ist.

8. Verfahren nach den Ansprüchen 1 bis 5 oder 7, **dadurch gekennzeichnet, dass** die Stützstruktur (2) eines der folgenden Materialien umfasst oder aus einem der folgenden Materialien gebildet ist: eine Mg-Legierung; eine Mg-Al-Zn-Legierung; eine Mg-Al-Mn-Legierung; eine Mg-Al-Zn-Mn-Legierung; eine Mg-RE-Legierung, wobei RE aus der Gruppe der Seltenen Erden ausgewählt ist; eine Mg-Y-RE-Legierung, wobei RE aus der Gruppe der Seltenen Erden ausgewählt ist; eine Mg-RE-Zn-Legierung, wobei RE aus der Gruppe der Seltenen Erden ausgewählt ist, eine Mg-Al-Y-Legierung; eine Mg-Al-RE-Legierung, wobei RE aus der Gruppe der Seltenen Erden ausgewählt ist; eine Mg-Zn-Zr-Legierung, eine Mg-Ca-Zn-Legierung; eine Mg-Al-Legierung mit einem Al-Gehalt von 0,01 Gew.-% bis 12 Gew.-%, vorzugsweise von 0,1 Gew.-% bis 5 Gew.-%, und einem Ca-Gehalt von 0,01 Gew.-% bis 5 Gew.-%, vorzugsweise von 0,1 Gew.-% bis 1 Gew.-%; eine Mg-Y-RE-Legierung, wobei RE für andere Seltene Erden steht, die sich von Y unterscheiden, mit einem Y-Gehalt von 0,1 Gew.-% bis 5 Gew.-%, einem Nd-Gehalt von 0,01 Gew.-% bis 5 Gew.-%, einem Gd-Gehalt von 0,01 Gew.-% bis 3 Gew.-%, einem Dy-Gehalt von 0,01 Gew.-% bis 3 Gew.-% und wobei die Legierung optional 0,1 Gew.-% bis 1 Gew.-% Zr und andere Seltenerdmetalle enthalten kann.

9. Verfahren nach einem der Ansprüche 1 bis 5, **dadurch gekennzeichnet, dass** die Stützstruktur (2) eine biologisch abbaubare polymere Stützstruktur ist, die vorzugsweise eines der folgenden Materialien umfasst oder aus einem der folgenden Materialien gebildet ist: ein biologisch abbaubares Polymer; ein Poly-L-Lactid; ein Poly-D,L-Lactid; ein Poly-L-Lactid-co-D,L-Lactid; ein Polyglycolid; ein Polyanhydrid; ein Polyhydroxybutyrat; ein Polyhydroxyvalerat; ein Poly-ε-Caprolacton; ein Polydioxanon; ein Poly(Lactid-co-Glycolid); ein Poly(Lactid-co-Caprolacton); ein Poly(Ethylenglycol-co-Caprolacton); ein Poly(Glycolid-co-Caprolacton); ein Poly(Hydroxybutyrat-co-Valerat); ein Polymer auf Polytrimethylencarbonatbasis; ein Polypropylensuccinat; ein Polyphosphazen; ein Poly-D,L-lactid-co-glycolid mit einem Lactidgehalt von 5 Gew.-% bis 85 Gew.-%, vorzugsweise von 50 Gew.-% bis 85 Gew.-%.

10. Verfahren nach einem der vorhergehenden Ansprüche, **dadurch gekennzeichnet, dass** die Polymerlösung (10) während des Elektrospinnens aus einer Düse (101) in Faserform auf die Stützstruktur (2) aufgebracht wird, vorzugsweise mit einem Volumenstrom der Polymerlösung (10) im Bereich von 0,5 ml/h bis 0,9 ml/h, vorzugsweise 0,7 ml/h, wobei die Düse (101) vorzugsweise einen Abstand (A) von der Stützstruktur (2) im Bereich von 70 mm bis 110 mm, vorzugsweise 90 mm, aufweist und zwischen der Düse (2) und einem Kollektor (200), auf dem die Stützstruktur (2) angeordnet ist, eine elektrische Spannung von wenigstens 1 kV, vorzugsweise von 1 kV bis 20 kV, besonders bevorzugt von 2 kV bis 10 kV, vorzugsweise 4 kV angelegt wird.

11. Verfahren nach einem der vorhergehenden Ansprüche, **dadurch gekennzeichnet, dass** nach dem Elektrospinnen die Hülle (3) bei einer vordefinierten Temperatur für einen vordefinierten Zeitraum temperiert wird, wobei der Zeitraum vorzugsweise im Bereich von 10 h bis 15 h liegt, der Zeitraum vorzugsweise 13,5 h beträgt und die Temperatur vorzugsweise im Bereich von 70 °C bis 90 °C liegt, wobei die Temperatur vorzugsweise 80 °C beträgt.

12. Intraluminale Endoprothese (1), hergestellt nach dem Verfahren gemäß einem der vorhergehenden Ansprüche.

## Revendications

1. Procédé de production d'une endoprothèse intraluminale (1), dans lequel l'endoprothèse (1) comprend une structure de support (2) et une gaine (3) agencée sur la structure de support (2), et dans lequel le procédé comprend les étapes consistant à :
- fournir la structure de support (2), et
- former la gaine (3), à partir de fibres polymères (30), sur la structure de support (2),
une solution de polymères (10) étant distribuée depuis une buse (101) par électrofilage, la solution de polymères (10) comprenant au moins un polymère biodégradable et au moins un additif,
**caractérisé en ce que**
le polymère biodégradable, au moins au nombre de un, est le poly-L-lactide, la solution de polymères (10) comprenant en outre comme additif de la 1,3-dioxan-2-one, la proportion de 1,3-dioxan-2-one parmi les substances dissoutes dans la solution de polymères (10) se trouvant dans la plage de 5 % en poids à 25 % en poids, sur la base de la masse totale des substances dissoutes, la proportion restante des substances dissoutes étant de préférence formée par du poly-L-lactide.

2. Procédé selon la revendication 1, **caractérisé en ce que** l'endoprothèse intraluminale (1) est un stent.

3. Procédé selon l'une des revendications précédentes, **caractérisé en ce que** le polymère biodégradable, au moins au nombre de un, est le poly-L-lactide, la solution de polymères (10) comprenant en outre comme additif de la 1,3-dioxan-2-one, la proportion de 1,3-dioxan-2-one parmi les substances dissoutes dans la solution de polymères (10) se trouvant dans la plage de 10 % en poids à 20 % en poids, de préférence de 10 % en poids à 15 % en poids, sur la base de la masse totale des substances dissoutes, la proportion restante des substances dissoutes étant de préférence formée par le poly-L-lactide.

4. Procédé selon la revendication 3, **caractérisé en ce que** les substances dissoutes ensemble dans la solution de polymères (10) présentent une concentration dans la plage de 1 % en poids à 10 % en poids, de préférence de 2 % en poids à 8 % en poids, de manière particulièrement préférée de 3 % en poids à 5 % en poids, de préférence égale à 4 % en poids.

5. Procédé selon l'une des revendications précédentes, **caractérisé en ce que** la solution de polymères (10) comprend un solvant, le solvant étant choisi dans le groupe constitué par : le chloroforme (CHCl₃), le trifluoroéthanol (TFE), un mélange comprenant du chloroforme et du trifluoroéthanol, le chloroforme et le trifluoroéthanol étant de préférence présents selon un rapport de mélange de 1:4.

6. Procédé selon l'une des revendications précédentes, **caractérisé en ce que** la structure de support (2) est une structure de support permanente, constituée de préférence de l'un des matériaux suivants ou comprenant au moins l'un des matériaux suivants : un alliage à base de Co-Cr, un alliage à base de Ni, un acier inoxydable résistant à la corrosion, un alliage de Ni-Ti, un alliage à base de Ti, un alliage à base de Nb, un alliage à base de Ta.

7. Procédé selon l'une des revendications 1 à 6, **caractérisé en ce que** la structure de support (2) est une structure de support métallique biodégradable.

8. Procédé selon les revendications 1 à 5 ou 7, **caractérisé en ce que** la structure de support (2) comprend l'un des matériaux suivants ou est formée à partir de l'un des matériaux suivants : un alliage de Mg ; un alliage de Mg-Al-Zn ; un alliage de Mg-Al-Mn ; un alliage de Mg-Al-Zn-Mn ; un alliage de Mg-RE, RE étant sélectionné dans le groupe des terres rares ; un alliage de Mg-Y-RE, RE étant sélectionné dans le groupe des terres rares ; un alliage de Mg-RE-Zn, RE étant sélectionné dans le groupe des terres rares ; un alliage de Mg-Al-Y ; un alliage de Mg-Al-RE, RE étant sélectionné dans le groupe des terres rares ; un alliage de Mg-Zn-Zr ; un alliage de Mg-Ca-Zn ; un alliage de Mg-Al présentant une teneur en Al de 0,01 % en poids à 12 % en poids, de préférence de 0,1 % en poids à 5 % en poids, et une teneur en Ca de 0,01 % en poids à 5 % en poids, de préférence de 0,1 % en poids à 1 % en poids ; un alliage de Mg-Y-RE, RE représentant d'autres terres rares différentes de Y, présentant une teneur en Y de 0,1 % en poids à 5 % en poids, une teneur en Nd de 0,01 % en poids à 5 % en poids, une teneur en Gd de 0,01 % en poids à 3 % en poids, une teneur en Dy de 0,01 % en poids à 3 % en poids, et l'alliage comprenant en option 0,1 % en poids à 1 % en poids de Zr et d'autres terres rares.

9. Procédé selon l'une des revendications 1 à 5, **caractérisé en ce que** la structure de support (2) est une structure de support polymère biodégradable, comprenant de préférence l'un des matériaux suivants ou étant formée à partir de l'un des matériaux suivants : un polymère biodégradable ; un poly-L-lactide ; un poly-D,L-lactide ; un poly-L-lactide-co-D,L-lactide ; un polyglycolide ; un polyanhydride ; un polyhydroxybutyrate ; un polyhydroxyvalérate ; une poly-ε-caprolactone ; une polydioxanone ; un poly(lactide-co-glycolide) ; une poly(lactide-co-caprolactone) ; une poly(éthylèneglycol-co-caprolactone) ; une poly(glycolide-co-caprolactone) ; un poly(hydroxybutyrate-co-valérate) ; un polymère à base de carbonate de polytriméthylène ; un succinate de polypropylène ; un polyphosphazène ; un poly-D,L-lactide-co-glycolide présentant une teneur en lactide de 5 % en poids à 85 % en poids, de préférence de 50 % en poids à 85 % en poids.

10. Procédé selon l'une des revendications précédentes, **caractérisé en ce que** la solution de polymères (10) est distribuée à partir d'une buse (101) pendant l'électrofilage sur la structure de support (2) sous forme de fibres, de préférence avec un écoulement volumique de la solution de polymères (10) dans la plage de 0,5 mL/h à 0,9 mL/h, de préférence égal à 0,7 mL/h, la buse (101) présentant de préférence une distance (A) par rapport à la structure de support (2) dans la plage de 70 mm à 110 mm, de préférence égale à 90 mm, et une tension électrique d'au moins 1 kV, de préférence de 1 kV à 20 kV, de manière particulièrement préférée de 2 kV à 10 kV, de préférence égale à 4 kV, et étant de préférence appliquée entre la buse (2) et un collecteur (200) sur lequel la structure de support (2) est agencée.

11. Procédé selon l'une des revendications précédentes, **caractérisé en ce qu'**après l'électrofilage, la gaine (3) est trempée à une température prédéfinie pendant une durée prédéfinie, la durée étant de préférence comprise entre 10 h et 15 h, la durée étant de préférence égale à 13,5 h, et la température étant de préférence comprise dans la plage de 70 °C à 90 °C, la température étant de préférence de 80 °C.

12. Endoprothèse intraluminale (1) produite par le procédé selon l'une des revendications précédentes.
